## Europäisches Patentamt

## European Patent Office

## Office européen des brevets

(19)

(11) Veröffentlichungsnummer: **0 049 847**
**B1**

(12) **EUROPÄISCHE PATENTSCHRIFT**

(45) Veröffentlichungstag der Patentschrift:
20.03.85

(21) Anmeldenummer: 81107960.7

(22) Anmeldetag: 06.10.81

(51) Int. Cl.⁴: **C 07 K 7/10**, C 07 K 1/14,
A 61 K 37/64, C 12 P 21/02 //
(C12P21/02, C12R1/465)

(54) **Alpha-Amylaseinaktivator, Verfahren zu seiner Herstellung, Mittel auf Basis dieses Inaktivators sowie dieser Inaktivator zur Regulierung des Blutzuckerspiegels.**

(30) Priorität: 09.10.80 DE 3038130
26.02.81 DE 3107106

(43) Veröffentlichungstag der Anmeldung:
21.04.82 Patentblatt 82/16

(45) Bekanntmachung des Hinweises auf die Patenterteilung:
20.03.85 Patentblatt 85/12

(84) Benannte Vertragsstaaten:
AT BE CH DE FR GB LI NL SE

(56) Entgegenhaltungen:
DE - A - 2 701 890

CHEMICAL ABSTRACTS, Band 95, Nr. 7, 17. August
1981, Seiten 261-262, Zusammenfassung 57064q,
COLUMBUS OHIO (US)

(73) Patentinhaber: HOECHST AKTIENGESELLSCHAFT,
Postfach 80 03 20, D-6230 Frankfurt am Main 80 (DE)

(72) Erfinder: **Vértesy, László, Dr., Eppenhainer Weg 6,
D-6239 Eppstein/Taunus (DE)**
Erfinder: **Mracek, Miroslav, Ulmenstrasse 45,
D-6000 Frankfurt am Main (DE)**
Erfinder: **Braunitzer, Gerhard, Prof. Dr.,
Schrämelstrasse 66, D-8000 München 60 (DE)**
Erfinder: **Aschauer, Heinz, Dr., Steindorfstrasse 1a,
D-8000 München 22 (DE)**

ACTORUM AG

## Beschreibung

In der DE-OS 2 701 890 wird ein α-Amylaseinhibitor beschrieben, der durch Fermentation von Streptomyces tendae 4158 (ATCC 31210) sowie seiner Varianten und Mutanten gewonnen wird. Er ist seiner chemischen Struktur nach ein Peptid und besitzt die Fähigkeit α-Amylase irreversibel zu inaktivieren. Aufgrund dieser Eigenschaften kann er zur Regulierung des Blutzuckerspiegels verwendet werden.

Es wurde nun gefunden, dass durch Anwendung eines verbesserten Verfahrens zur Gewinnung und Reinigung des α-Amylaseinaktivators, im folgenden auch als HOE 467 bezeichnet, der Inaktivator auf einfacherem Wege in grösserer Reinheit hergestellt werden kann, und dass die hochreine Substanz aus 2 Komponenten, im folgenden als HOE 467-A und HOE 467-B bezeichnet, besteht.

Sofern nichts anderes gesagt ist, werden in den vorstehenden und folgenden Ausführungen unter α-Amylaseinaktivator sowohl das Gemisch als auch die Einzelkomponenten verstanden.

Die Erfindung betrifft daher einen α-Amylaseinaktivator bestehend aus den Komponenten HOE 467-A und HOE 467-B, oder aus der Komponente HOE 467-A oder HOE 467-B sowie ein Verfahren zu seiner Herstellung und Auftrennung in die beiden Komponenten.

Für den erfindungsgemässen α-Amylaseinaktivators HOE 467 sowie für die beiden Einzelkomponenten HOE 467-A und HOE 467-B trifft die in der DE-OS 2 701 890 angegebene Substanzbeschreibung mit Ausnahme der folgenden wesentlichen Unterschiede zu:

Er besitzt eine erhöhte enzymatische Wirksamkeit und besteht aus den 2 Komponenten HOE 467-A und HOE 467-B, die durch folgende Daten charakterisiert sind:

Das Hydrolysat von HOE 467-A weist folgende Aminosäurezusammensetzung auf:

| Asp 6 | Glu 7 | Ala 7 | | Tyr 6 | Lys 1 |
| Thr 8 | Pro 3 | Val 8 | Ile 2 | | Arg 3 |
| Ser 5 | Gly 7 | Cys 4 | Leu 4 | His 2 | Trp 1 |

Das Hydrolysat von HOE 467-B hat folgende Aminosäurezusammensetzung:

| Asp 5 | Glu 6–7 | Ala 7 | | Tyr 6 | Lys 1 |
| Thr 6–8 | Pro 3 | Val 7–8 | Ile 2 | | Arg 3 |
| Ser 4–5 | Gly 7 | Cys 4 | Leu 4 | His 2 | Trp 1 |

Die Bestimmung von Trp erfolgte durch Absorptionsmessung im UV-Licht. Die übrigen Aminosäuren wurden nach hydrolytischer Spaltung bestimmt.

Die isoelektrischen Punkte der beiden Komponenten unterscheiden sich geringfügig, sie sind abhängig von der Ionenstärke sowie den Bestimmungsmethoden (Biochemisches Taschenbuch, herausgegeben von H.M. Rauen, Springer Verlag, 1964). Die durch isoelektrische Fokussierung (R.C. Allen, H.R. Maurer: Elektrophoresis and Isoelectric Focusing in Polyacrylamid Gel, W. de Gruyter, Berlin, 1974) erhaltenen Werte betragen:

HOE 467-A:   4,35 ± 0,15
HOE 467-B:   4,53 ± 0,15

Der erfindungsgemässe hochreine α-Amylaseinaktivator HOE 467-A ist ferner dadurch gekennzeichnet, dass seine Endgruppe die Asparaginsäure ist, dass er aus 74 Aminosäuren mit folgender Sequenz

Asp-Thr-Thr-Val-Ser-Glu-Pro-Ala-Pro-Ser-Cys-Val-Thr-Leu-Tyr-Gln-Ser-Trp-Arg-Tyr-Ser-Gln-Ala-Asp-Asn-Gly-Cys-Ala-Glu-Thr-Val-Thr-Val-Lys-Val-Val-Tyr-Glu-Asp-Asp-Thr-Glu-Gly-Leu-Cys-Tyr-Ala-Val-Ala-Pro-Gly-Gln-Ile-Thr-Thr-Val-Gly-Asp-Gly-Tyr-Ile-Gly-Ser-His-Gly-His-Ala-Arg-Tyr-Leu-Ala-Arg-Cys-Leu

besteht und zwischen Cys 11 und Cys 27 sowie zwischen Cys 45 und Cys 73 Disulfidbrücken aufweist. Sein Molekulargewicht berechnet sich aufgrund der Zusammensetzung zu 7958.

Zur Bestimmung der Aminosäurezusammensetzung wurde HOE 467-A hydrolytisch gespalten, wobei aus Glutamin (Gln) und Asparagin (Asn) die entsprechenden Säuren Glu und Asp entstanden.

Der Inaktivator HOE 467-B ist ein am N-terminalen Kettenende verkürztes Abbauprodukt von HOE 467-A. So kann z.B. bei einem der möglichen Abbauprodukte Serin die terminale Gruppe sein.

Für die beiden Komponenten HOE 467-A und HOE 467-B beträgt die spezifische Hemmwirkung gegen die α-Amylase aus Schweinepankreas jeweils $1,7 \cdot 10^7$ AIE/g.

Die Hemmwirkung wurde in dem nachfolgend beschriebenen Amylasetest bestimmt:

Amylasetest

Eine Amylase-Inhibitoreinheit (AIE) ist definiert als die Inhibitormenge, die unter den Testbedingungen zwei Amylaseeinheiten (AE) zu 50% zu hemmen vermag. Eine Amylaseeinheit ist nach internationaler Übereinkunft die Enzymmenge, die in einer Minute 1 μ Äquivalent glucosidischer Bindungen in der Stärke spaltet. Die μVal gespaltener glucosidischer Bindungen werden als μVal reduzierender Zucker photometrisch mit Dinitrosalizylsäure bestimmt. Die Angaben sind als μMole Maltose berechnet, die anhand einer Maltose-Eichgeraden ermittelt werden.

Die Tests werden folgendermassen durchgeführt:

α-Amylase aus Schweinepankreas und die zu testende Lösung werden gemeinsam in 1,0 ml 20 mM Phosphatpuffer, pH 6,9 + 10 mM NaCl 10–20 min. bei 37°C vorinkubiert. Die enzymatische Reaktion wird durch Zugabe von 1,0 ml löslicher Stärke nach Zulkowski (0,25% in dem angegebenen Puffer) gestartet. Nach genau 10 min. wird die Reakton mit 2,0 ml Dinitrosalizylsäure-Farbreagenz (nach Boehringer Mannheim: Biochemica-Information II) abgestoppt und zur Farbentwicklung 5 min. im siedenden Wasserbad erhitzt. Nach dem Abkühlen wird die Extinktion bei 546 nm gegen den Reagentienleerwert gemessen. Die 50%ige Hemmung wird im Vergleich zur ungehemmten Enzymreaktion durch Einsatz verschiedener Inhibitormengen graphisch mittels der Wahrscheinlichkeitsauftragung bestimmt.

Bei der Herstellung des erfindungsgemässen α-Amylaseinaktivators bedient man sich erfindungsgemäss des Stammes Streptomyces tendae 4158 (ATCC 31210) oder des abgeleiteten Stammes Streptomyces tendae HAG 1266 (DSM 1912). Der Inaktivator wird aus der Kulturlösung gewonnen. Die Anwendung des Stammes Streptomyces tendae HAG 1266 ist bevorzugt.

Die Erfindung betrifft ferner auch den Stamm Streptomyces tendae HAG 1266 (DSM 1912).

Der neue Stamm unterscheidet sich von dem Stamm Streptomyces tendae ATCC 31210 durch wichtige Eigenschaften. Auffällig sind die unterschiedlichen morphologischen Charakteristika beider Stämme und ebenso neue, für die fermentative Herstellung und die Isolierung des erfindungsgemässen α-Amylaseinaktivators bedeutende Eigenschaften, wie das schnellere Wachstum und die um etwa 40% erhöhte α-Amylase-Inaktivator-Produktionskapazität. Die Melanin-Bildung, die sich bei dem Streptomyces tendae ATTC 31210 durch eine braun-schwarze Farbe der Kulturlösung und des Kulturfiltrats manifestiert und die Aufarbeitung vor gewisse Reinigungsprobleme stellt, wurde bei dem neuen Stamm HAG 1266 (DSM 1912) mit Hilfe von genetischen Eingriffen auf etwa 1/10 reduziert und auch qualitativ durch eine farbliche Verschiebung verändert. Die Melaninpigmente zeigen nun ein gelb-rötliches Farbspektrum. Die Eliminierung dieser Melaninart bei der Aufarbeitung ist unproblematisch.

Die beiden α-Amylase-Inaktivator produzierenden Stämme sind in der folgenden Tabelle gegenübergestellt:

Tabelle
Eigenschaften der Stämme ATCC 31210 und HAG 1266

|  |  | ATCC 31210 | HAG 1266 (DSM 1912) |
|---|---|---|---|
| Farbe des Substratmycels |  | gelb-braun | rötlich |
| Farbe des versporten Luftmycels |  | grau-braun | weiss-gelb |
| Morphologie der Sporenketten |  | Retinaculum apertum (RA) | Flexibilis (F) |
| Sporenmorphologie |  | kugelförmig, leicht warzig bis glatt ~Grösse ∅ 1 μm | kugelförmig, scharfkantige Warzen ~Grösse ∅ 1,3 μm |
| Melaninbildung auf Peptonmedium |  | positiv | negativ |
| Nitratreduktion |  | positiv | positiv |
| Substrat- verwertungs- spektrum | Glukose | + + | + + + |
|  | Fruktose | ± | + + |
|  | Saccharose | + | + + + |
|  | Maltose | + | + |
|  | Lactose | ± | − |
|  | Galaktose | + | − |
|  | Rhamnose | ± | − |
|  | Sorbose | − | − |
|  | Xylose | + | − |
|  | Arabinose | + | + + |
|  | Inositol | + + | − |
|  | Manitol | + + | − |
|  | Raffinose | ± | − |
|  | Cellulose | − | − |

Der Stamm Streptomyces tendae HAG 1266 ist bei der Deutschen Sammlung von Mikroorganismen (DSM) unter der Registriernummer DSM 1912 hinterlegt worden.

Die Fermentation wird zweckmässig in analoger Weise wie in der DE-OS 2 701 890 beschrieben, durchgeführt.

Die fertigen Fermentationslösungen des Strep-

tomyces tendae ATCC 31210 und HAG 1266 enthalten Enzyme, die die Wirkstoffkonzentration während der Aufarbeitung beträchtlich vermindern können. Darüberhinaus können Stoffe wie z.B. Melanin vorhanden sein, deren vollständige Abtrennung mit den bekannten Verfahren nur schwierig zu erreichen ist.

Es ist nun gefunden worden, dass die erforderlichen Abtrennungen des Inaktivators aus der Kulturlösung durch Verwendung von sogenannten Adsorptionsharzen (DE-PS 1 274 128, US-PS 3 531 963) leicht durchgeführt werden können.

Das erfindungsgemässe Verfahren zur Herstellung des α-Amylaseinaktivators ist dadurch gekennzeichnet, dass man Streptomyces tendae ATCC 31210 oder 1266 kultiviert und aus der Kultur den Inaktivator mit Hilfe von Adsorptionsharzen oder Reversephase-Chromatographie abtrennt und anschliessend reinigt.

Geeignet sind handelsübliche Harze wie z.B. Polystyrolharze. Zur Anwendung wird das Harz mit dem Kulturfiltrat in Berührung gebracht. Der pH-Wert der Kulturflüssigkeiten wird auf 2 bis 8, vorzugsweise 4 bis 6, eingestellt. Das feste Harz adsorbiert den Inaktivator HOE 467 selektiv, wohingegen die unerwünschten Enzyme sowie der grösste Teil der Verunreinigungen durch Filtration entfernt werden können, da sie ungebunden in Lösung bleiben. Die Wiedergewinnung des HOE 467 vom Harz erfolgt zweckmässigerweise entweder durch Waschen mit geeigneten wässrigen Pufferlösungen wie z.B. Phosphatpuffer oder mit wässrigen Lösungen von organischen Lösungsmitteln wie z.B. niederen aliphatischen Alkoholen, Aceton, Acetonitril oder anderen. Die weitere Reinigung des nun angereicherten Inaktivators HOE 467 erfolgt nach bekannten Verfahren.

Die geschilderte Trennung mit Adsorptionsharzen beruht auf dem Prinzip der Verteilung verschiedener polarer Verbindungen. Für die erforderliche rasche Abtrennung lassen sich noch weitere Methoden der Verteilung verwenden. So sind ferner Verteilungen im Sinne der Reversephase-Chromatographie geeignet (G. Schwendt, Chromatographische Trennmethoden, Georg Thieme Verlag, Stuttgart, 1979), wobei entweder käufliche Träger oder selbst hergestellte, z.B. silanisierte Träger, Verwendung finden. Schliesslich sei auch noch auf die Flüssig-flüssig-Trennverfahren hingewiesen, z.B. mit wässrigem Polyäthylenglykol, wässrigen Phosphatpuffersystemen, wie sie im Prinzip beispielsweise in der DE-OS 2 639 129 beschrieben sind.

Es ist in der DE-OS 2 701 890 beschrieben worden, dass sich Ionenaustauscher, wie z.B. DEAE-Cellulose zur Reinigung des α-Amylaseinaktivators HOE 467 eignen. Hierbei wurden – wie in der Ionenaustauscherchromatographie üblich – pH-Werte gewählt, die einen erforderlichen Ionisierungsgrad gewährleisten. Es wurde nun überraschend gefunden, dass wenn man Reinigungen – entgegen der Regel – in der Nähe des isoelektrischen Punktes, d.h. bei einem geringen Ionisierungsgrad durchführt, HOE 467 in zwei Komponenten getrennt wird. Geeignet ist zur Trennung der pH-Bereich von 4,4–6, vorzugsweise von 4,8 bis 5,3. Dabei wird eine Komponente des Wirkstoffs vom Ionenaustauscher festgehalten und kann von diesem nur durch Änderung des pH-Wertes oder durch Erhöhen der Salzkonzentration heruntergelöst werden. Dieser Stoff wird HOE 467-A genannt. Die andere Komponente bleibt unter den genannten Bedingungen vom Ionenaustauscher mehr oder weniger ungebunden und kann soweit leicht vom Träger gewaschen werden. Diese Komponente wird HOE 467-B genannt.

Obwohl die Komponenten für eine pharmazeutische Anwendung hinreichend rein anfallen, kann noch eine zusätzliche Reinigung durchgeführt werden.

So wird der Inaktivator HOE 467-A z.B. durch Chromatographie, in An- oder Abwesenheit eines dissoziierenden Puffers, vorzugsweise Harnstoff (6–8 M wässrige Lösung), über DEAE- oder Kationenaustauscher-Säulen, wie z.B. Carboxymethylcellulose-Säulen, gereinigt und nach üblichen Methoden isoliert. Diese zusätzliche Reinigung wird z.B. vor Bestimmung der Aminosäuresequenz durchgeführt.

Der erfindungsgemässe α-Amylaseinaktivators ist gegenüber enzymatischem Abbau resistent. Seine Eigenschaften sind im Hinblick auf seine Anwendung als Arzneimittel, insbesondere als Therapeutikum gegen Diabetes und Prädiabetes sowie Adipositas, sowie auf seine Anwendung zur Unterstützung von Diät interessant. Bei der Anwendung können sowohl die Einzelkomponenten als auch das Gemisch aus HOE 467-A und HOE 467-B eingesetzt werden.

Die Erfindung betrifft daher auch ein pharmazeutisches Mittel enthaltend den erfindungsgemässen Amylaseinaktivator sowie seine Verwendung.

Stärkehaltige Nahrungs- und Genussmittel führen bei Tier und Mensch zu einem Anstieg des Blutzuckers und dadurch auch zu einer vermehrten Insulinsekretion des Pankreas. Die Hyperglykämie kommt durch die Aufspaltung der Stärke im Verdauungstrakt unter Einwirkung von Amylase und Maltase zu Glukose zustande.

Bei Diabetikern ist diese Hyperglykämie besonders ausgeprägt und langanhaltend.

Bei Adipösen wirkt die vermehrte Insulinsekretion auf die Lipogenese und vermindert die Lipolyse.

Die alimentäre Hyperglykämie sowie die Hyperinsulinämie nach Stärkeaufnahme lässt sich durch den beanspruchten Amylase-Inaktivator vermindern. Diese Wirkung ist dosisabhängig. Der erfindungsgemässe Amylase-Inaktivator lässt sich daher als Therapeuticum einsetzen bei Diabetes, Prädiabetes und Adipositas sowie zur Unterstützung von Diät. Zu diesem Zweck empfiehlt sich eine Verabreichung, insbesondere zu den Mahlzeiten. Die Dosierung, die sich nach dem Gewicht des Patienten sowie dem individuellen Bedarf ausrichten soll, beträgt etwa 10 000

bis 300 000 AIE, sie kann jedoch in begründeten Fällen auch darüber oder darunter liegen.

Der erfindungsgemässe Amylase-Inaktivator eignet sich insbesondere zur oralen Verabreichung. Er kann als reine Substanz aber auch in Form einer pharmazeutischen Zubereitung unter Verwendung der üblichen Hilfs- und Trägerstoffe angewandt werden, wie z.B. Talkum, Magnesiumstearat, Milchzucker, Stärke oder Polyethylenglykole. Als Darreichungsform eignen sich beispielsweise Tabletten, Steckkapseln oder auch ein Granulat.

Auch eine kombinierte Anwendung mit anderen Arzneimitteln wie blutzuckersenkenden oder lipidsenkenden Substanzen kann von Vorteil sein.

Da höhermolekulare Peptide als solche nicht oder nicht nennenswert aus dem Verdauungstrakt resorbiert werden, sind von der erfindungsgemässen Substanz keine toxikologisch bedenklichen Nebenwirkungen zu erwarten. Aufgrund der nicht aussergewöhnlichen Aminosäurezusammensetzung sind auch evtl. proteolytische Spaltprodukte als physiologisch unbedenklich anzusehen. Dementsprechend konnten bei oraler Gabe auch hoher Dosen des Amylaseinaktivators an Versuchstiere keine auffälligen Symptome erkannt werden. Auch bei intravenöser Applikation an Mäusen (1 g/kg) wurde der erfindungsgemässe Inaktivator bei 24stündiger Beobachtungszeit ohne erkennbare toxische Wirkung vertragen.

Zur Prüfung der pharmakologischen Wirkung des Amylase-Inaktivators erhielten nüchterne, männliche Wistar-Ratten mit einem Gewicht zwischen 200 und 250 g den erfindungsgemässen Inaktivator gleichzeitig mit 2 g Stärke pro kg Körpergewicht oral verabreicht, nachdem unmittelbar zuvor eine Blutentnahme zur Bestimmung des Ausgangsblutzuckerwertes erfolgte. Weitere Blutentnahmen erfolgten nach 15 und 30 Minuten sowie nach 1, 2, 3 und 5 Stunden aus der Schwanzvene. Die Blutzuckerbestimmungen wurden nach der Methode von Hoffman im Autoanalyzer [J. biol. Chem. 120, 51 (1937)] durchgeführt.

NZO-Mäuse weisen eine gestörte Glucosetoleranz auf. Sie eignen sich deshalb besonders gut für Untersuchungen, bei denen der Blutglucosespiegel beeinflusst wird. Die Versuchsanordnung entspricht der bei Ratten. Die Blutentnahme erfolgt aus dem Orbitalvenen-Plexus. Der Blutzuckerverlauf wird über 3 Stunden verfolgt.

In anologer Weise wird der Wirkstoffnachweis an NMRI-Mäusen erbracht. Die Blutentnahmen erfolgen ebenfalls aus dem Orbitalvenen-Plexus und der Blutzuckerverlauf wird über 3 Stunden verfolgt.

Unter diesen Versuchsanordnungen zeigten die mit dem erfindungsgemässen Inaktivator behandelten Tiere einen gegenüber unbehandelten Tieren geringeren, protrahierten Blutzuckeranstieg.

Beispiel 1

Der Stamm Streptomyces tendae HAG 1266 wird auf Schrägröhrchen mit einem Nährboden folgender Zusammensetzung geimpft:

Haferflocken 50 g
ad. 1000 ml $H_2O$
pH 7,2

Das beimpfte Röhrchen wird 10 Tage bei 28°C bebrütet und danach bei +4°C gelagert. Die aus dem gelblichen Luftmyzel sich leicht lösenden Sporen werden in 10 ml sterilisiertes aqua dest. mit 0,2 ml Tween 80 suspendiert. $10^8$–$10^9$-Sporen, d.h. 1 ml der Suspension dient zur Beimpfung eines 300 ml Erlenmeyerkolbens, der mit 35 ml sterilisierter Nährlösung mit einem pH-Wert von 7,2 und folgender Zusammensetzung beschickt ist:

1% Glukose
1% Sojamehl
0,25% NaCl

Die Sterilisationszeit beträgt 45 min. bei 121°C und 1 bar. Der Kolben wird bei 250 Upm 40 Stunden bei +30°C auf einer Schüttelmaschine bei einer Amplitude 3,5 cm geschüttelt. Je 5 ml dieser Vorkultur werden in mehrere Erlenmeyerkolben überführt, die mit 50 ml sterilisierter Nährlösung beschickt sind und einen pH-Wert von 7,4 haben. Die Zusammensetzung dieser Hauptkultur ist folgende:

| | |
|---|---|
| Stärke löslich | 4 % |
| Sojamehl | 0,4% |
| Cornsteep flüssig | 0,4% |
| Magermilchpulver | 0,7% |
| Glukose | 1,0% |
| $(NH_4)_2HPO_4$ | 1,2% |

Die Sterilisationszeit beträgt 45 min. bei 121°C und 1 bar. Die Hauptkulturen wurden bei 25°C mit 250 Upm auf einer Schüttelmaschine mit einer Amplitude von 3,5 cm 96–120 Stunden geschüttelt. Am dritten, vierten und fünften Kulturtag wird der Gehalt an α-Amylase-Inaktivator gemäss Testvorschrift bestimmt.

Der Stamm Streptomyces tendae HAG 1266 liefert unter den beschriebenen Versuchs- und Kulturbedingungen bei einem End-pH von 6,4 1900 ATE/ml.

Beispiel 2

Ansatz wie im Beispiel 1, die Hauptfermentation wird jedoch in einem Fermenter von 15 l Gesamtvolumen mit Füllung 10 l durchgeführt. Es wird folgende Nährlösung-Zusammensetzung verwendet:

| | |
|---|---|
| Stärke | 4 % |
| Sojamehl | 0,4% |
| Cornsteep flüsig | 0,4% |
| Magermilchpulver | 0,7% |
| Glucose | 1,0% |
| $(NH_4)_2HPO_4$ | 1,2% |

Der pH-Wert soll nach der Sterilisation 6,8 betragen und wird nach Bedarf mit sterilisierter Säure (2n $H_3PO_4$) oder Lauge (2 n NaOH) auf diesen Wert eingestellt. Beimpft wird die Hauptstufe mit 1 l, entsprechend 10%, einer wie unter Beispiel 1 beschriebenen Vorkultur.

Es wird 50 bis 70 Std. bei 30°C fermentiert. Die Belüftung beträgt 300 l/h bei einer Rührung von 250 Upm und einem Überdruck von 0,3 bar.

Durch die Probenahme wird der Fermentationsverlauf hinsichtlich der Inhibitoraktivität, des Carbohydrateabbaus, der Biomasseentwicklung und des physikalisch-technischen Verhaltens der Kulturlösung (Oberflächenspannung, Viskosität, Dichte, osmotischer Druck) überwacht und verfolgt.

Die maximale Inhibitoraktivität wird ab der 60. Kulturstunde mit durchschnittlich 1800 AIE/ml erreicht. Danach wird der Fermenterinhalt der Aufarbeitung zugeführt.

Beispiel 3

6,5 l Kulturfiltrat, gewonnen durch Abnutschen der nach Beispiel 2 gewonnenen Streptomyceskultur, wurden mit Eisessig unter Rühren auf pH 4,9 gestellt und auf eine vorbereitete Glassäule aufgetragen. Die Säule beinhaltete 230 g Polystyrol-Adsorptionsharz (z.B. Diaion® HP 20) in Wasser aufgeschlämmt. Die Säulenmasse betrugen 22×5 cm entsprechend einem Volumen von 430 ml. Den Flüssigkeitsdurchfluss regelte man so, dass nach 2 Stunden 6,5 l durchgelaufen sind. Dann war der Adsorptionsvorgang beendet und das Harz konnte zunächst mit reinem Wasser gewaschen und anschliessend mit Wasser, dem steigende Mengen Isopropanol hinzugefügt worden sind, eluiert werden. Die durchfliessende Flüssigkeit wurde in Fraktionen von je 1 l eingefangen und auf enzymhemmende Wirkung getestet. Die aktiven Fraktionen wurden gesammelt und im Vakuum auf 100 ml destillativ eingeengt. Das Konzentrat enthielt 110 000 AIE/ml.

Die so entstandene, jetzt salzfreie Lösung wurde direkt auf eine mit ⅟₃₀-Phosphatpuffer, pH 7,5, vorbereitete DEAE-Ionenaustauscher-Säule aufgetragen und entsprechend DAS 2 701 890, Beispiel 5, gereinigt. Es resultierten 4 g Substanz mit 2500 AIE/mg.

Beispiel 4

Zur weiteren Reinigung wurde eine Glassäule von 2 cm Durchmesser und 22 cm Höhe, entsprechend 70 ml Inhalt, mit DEAE-Cellulose gefüllt, die vorher mit ⅟₁₀ m Natriumacetatpuffer, pH 4,9 und 0,02% Natriumazid equilibriert worden ist. Nun wurde die nach Beispiel 3 erhaltene Substanz in 10 ml des gleichen Puffers gelöst und auf die Säule aufgetragen. Zunächst wusch man den Säuleninhalt mit 100 ml Acetatpuffer, dann wurde zu diesem Elutionsmittel allmählich Kochsalz so zugemischt, dass ein kontinuierlicher Gradient gewährleistet war. Beim fraktionierten Auffangen des Säulendurchflusses befand sich HOE 467-B in den noch Kochsalz-freien Anfangsfraktionen, während die HOE 467-A Komponente

in den Fraktionen nachweisbar war, in denen die NaCl-Konzentration 0,25 bis 0,3 molar war. Die B- sowie die A-Komponenten enthaltenden Fraktionen wurden getrennt gesammelt, gegen destilliertes Wasser dialysiert und gefriergetrocknet. Die farblosen Substanzen hatten eine Wirksamkeit von jeweils

$1,7 \cdot 10^4$ AIE/mg.

Die Aminosäurenanalyse der Produkte ergab nach 20stündiger Salzsäurehydrolyse mit Hilfe eines Beckman-Multichroanalysators folgende prozentuale Zusammensetzungen:

|  | HOE 467-A | HOE 467-B |
|---|---|---|
| Asparginsäure | 8,51 | 7,90 |
| Threonin | 10,07 | 8,17 |
| Serin | 5,41 | 5,98 |
| Glutaminsäure | 11,30 | 12,39 |
| Prolin | 3,28 | 3,39 |
| Glycin | 5,61 | 6,19 |
| Alanin | 6,85 | 7,41 |
| Cystein | 4,68 | 3,83 |
| Valin | 8,69 | 8,54 |
| Methionin | – | – |
| Isoleucin | 2,93 | 3,28 |
| Leucin | 5,91 | 6,61 |
| Tyrosin | 11,28 | 12,69 |
| Phenylalanin | – | – |
| Histidin | 3,49 | 3,72 |
| Lysin | 1,78 | 2,05 |
| Arginin | 6,12 | 6,52 |

Beispiel 5

In analoger Weise erhält man aus der Kulturlösung von Streptomyces tendae ATCC 31210 den α-Amylaseinaktivator HOE 467-A und HOE 467-B.

Beispiel 6

a) Herstellung des N-terminal einheitlichen α-Amylaseinaktivators HOE 467-A:

HOE 467-A erhalten gemäss Beispiel 4 oder 5 wird durch Chromatographie über eine DEAE- oder Carboxymethylcellulose-Säule in Gegenwart von 8M, wässriger Harnstofflösung als dissoziierendem Puffer gereinigt und nach üblichen Methoden isoliert.

b) Herstellung tryptischer Peptide:

Der gemäss a) erhaltene α-Amylaseinaktivator HOE 467-A mit reiner Endgruppe wird zur Aufspaltung der Disulfidbrücken entweder mit Perameisensäure oxydiert oder mit Aziran umgesetzt [vgl. C.H.W. Hirs, J. Biol. Chem. 219, 611–621 (1956) und M.A. Raftery und R.D. Cole, J. Biol. Chem. 241, 3457–3461 (1966)] und nach üblichen Methoden tryptisch gespalten. Die tryptischen Hydrolysate werden chromatographisch getrennt und gereinigt.

c) Sequenzanalyse:

Die Sequenzanalyse wurde nach der Filmtechnik [P. Edmann und G. Begg, Eur. J. Biochem. 1, 80–91 (1967)] durchgeführt unter Anwendung folgender Programme:

α) Quadrolprogramm: Es wurde der gemäss b) mit Aziran umgesetzte α-Amylaseinaktivator HOE 467-A und ein limitiert tryptisch gespaltener Inaktivator A abgebaut.

β) Propinprogramm: Hier wurden die übrigen Peptide abgebaut [vgl. G. Braunitzer und B. Schrank, Hoppe-Seyler's Z. Physiol. Chem. 351, 88 (1970) und G. Braunitzer, A. Stangel und O. Scheithauer, Hoppe-Seyler's Z. Physiol. Chem. 359, 137–146 (1978)].

d) Aminosäureanalyse:
Sie wurde nach Hydrolyse des gemäss a) erhaltenen Inaktivators mit 6 N Salzsäure durchgeführt.

e) Bestimmung der Disulfidbrücken:
Hierzu wurde die gemäss a) erhaltene Substanz in verdünnter Ameisensäure mit Pepsin während 18 Stunden behandelt, die Spaltprodukte wurden nach üblichen Methoden gereinigt.
Aufgrund der durchgeführten Operationen zeigte sich, dass die gemäss a) erhaltene Substanz aus 74 Aminosäuren besteht, deren Sequenz wie folgt ist:
Asp-Thr-Thr-Val-Ser-Glu-Pro-Ala-Pro-Ser-Cys-
Val-Thr-Leu-Tyr-Gln-Ser-Trp-Arg-Tyr-Ser-Gln-
Ala-Asp-Asn-Gly-Cys-Ala-Glu-Thr-Val-Thr-Val-
Lys-Val-Val-Tyr-Glu-Asp-Asp-Thr-Glu-Gly-Leu-
Cys-Tyr-Ala-Val-Ala-Pro-Gly-Gln-Ile-Thr-Thr-
Val-Gly-Asp-Gly-Tyr-Ile-Gly-Ser-His-Gly-His-
Ala-Arg-Tyr-Leu-Ala-Arg-Cys-Leu
und die an den genannten Stellen Disulfidbrücken besitzt.

**Patentansprüche für die Vertragsstaaten:**
**BE, CH, DE, FR, GB, LI, NL, SE**

1. α-Amylaseinaktivator, bestehend aus dem Inaktivator HOE 467-A, dessen Hydrolysat folgende Aminosäurezusammensetzung aufweist:

| Asp 6 | Glu 7 | Ala 7 | | Tyr 6 | Lys 1 |
|-------|-------|-------|-------|-------|-------|
| Thr 8 | Pro 3 | Val 8 | Ile 2 | | Arg 3 |
| Ser 5 | Gly 7 | Cys 4 | Leu 4 | His 2 | Trp 1 |

und der einen isoelektrischen Punkt bei 4,35 ± 0,15 besitzt oder bestehend aus dem Inaktivator HOE 467-B, dessen Hydrolysat folgende Aminosäurezusammensetzung aufweist:

| Asp 5 | Glu 6–7 | Ala 7 | | Tyr 6 | Lys 1 |
|-------|---------|-------|-------|-------|-------|
| Thr 6–8 | Pro 3 | Val 7–8 | Ile 2 | | Arg 3 |
| Ser 4–5 | Gly 7 | Cys 4 | Leu 4 | His 2 | Trp 1 |

und der einen isoelektrischen Punkt bei 4,53 ± 0,15 besitzt oder bestehend aus den beiden Komponenten HOE 467-A und HOE 467-B.

2. α-Amylaseinaktivator gemäss Anspruch 1, dadurch gekennzeichnet, dass HOE 467-A ein Molekulargewicht von 7958 besitzt und dass die 74 Aminosäuren folgende Sequenz aufweisen:

Asp-Thr-Thr-Val-Ser-Glu-Pro-Ala-Pro-Ser-Cys-
Val-Thr-Leu-Tyr-Gln-Ser-Trp-Arg-Tyr-Ser-Gln-
Ala-Asp-Asn-Gly-Cys-Ala-Glu-Thr-Val-Thr-Val-
Lys-Val-Val-Tyr-Glu-Asp-Asp-Thr-Glu-Gly-Leu-
Cys-Tyr-Ala-Val-Ala-Pro-Gly-Gln-Ile-Thr-Thr-
Val-Gly-Asp-Gly-Tyr-Ile-Gly-Ser-His-Gly-His-
Ala-Arg-Tyr-Leu-Ala-Arg-Cys-Leu
und dass zwischen Cys 11 und Cys 27 sowie zwischen Cys 45 und Cys 73 Disulfidbrücken sind.

3. Verfahren zur Herstellung des α-Amylaseinaktivators gemäss Anspruch 1, dadurch gekennzeichnet, dass man Streptomyces tendae ATCC 31210 oder HAG 1266 kultiviert und aus der Kultur den Inaktivator HOE 467 mit Hilfe von Adsorptionsharzen oder Reversephase-Chromatographie abtrennt und anschliessend durch Ionenaustauscherchromatographie in einem pH-Bereich von 4,4 bis 6 in die zwei Komponenten HOE 467-A und HOE 467-B auftrennt.

4. Verfahren gemäss Anspruch 3, dadurch gekennzeichnet, dass man den abgetrennten Inaktivator HOE 467 mit Hilfe von Ionenaustauschern im pH-Bereich zwischen 4,8 und 5,3 in die beiden Komponenten HOE 467-A und HOE 467-B auftrennt.

5. Verfahren gemäss Anspruch 3, dadurch gekennzeichnet, dass man den abgetrennten Inaktivator HOE 467 mit Hilfe von Ionenaustauschern im pH-Bereich zwischen 4,4 und 6 in die beiden Komponenten HOE 467-A und HOE 467-B auftrennt und HOE 467-A durch Chromatographie an DEAE- oder Carboxymethyl-cellulose-Säulen in Gegenwart eines dissoziierenden Puffers weiter reinigt und isoliert.

6. Streptomyces tendae HAG 1266 (DSM 1912).

7. Pharmazeutisches Präparat enthaltend den α-Amylaseinaktivator gemäss Anspruch 1.

8. α-Amylaseinaktivator gemäss Anspruch 1 zur Regulierung des Blutzuckerspiegels.

**Patentansprüche für den Vertragsstaat: AT**

1. Verfahren zur Herstellung eines α-Amylaseinaktivators, dadurch gekennzeichnet, dass man Streptomyces tendae ATCC 31210 oder DSM 1912 kultiviert und aus der Kultur den Inaktivator HOE 467 mit Hilfe von Adsorptionsharzen oder Reversephase-Chromatographie abtrennt und anschliessend durch Ionenaustauscherchromatographie in einem pH-Bereich von 4,4 bis 6 in zwei Komponenten auftrennt, wobei ein α-Amylaseinaktivator, bestehend aus HOE 467-A, dessen Hydrolysat folgende Aminosäurezusammensetzung aufweist:

| Asp 6 | Glu 7 | Ala 7 | | Tyr 6 | Lys 1 |
|-------|-------|-------|-------|-------|-------|
| Thr 8 | Pro 3 | Val 8 | Ile 2 | | Arg 3 |
| Ser 5 | Gly 7 | Cys 4 | Leu 4 | His 2 | Trp 1 |

und der einen isoelektrischen Punkt bei 4,35 ± 0,15 besitzt oder bestehend aus dem Inaktivator HOE 467-B, dessen Hydrolysat folgende Aminosäurezusammensetzung aufweist:

| Asp 5 | Glu 6–7 | Ala 7 | | Tyr 6 | Lys 1 |
|-------|---------|-------|-------|-------|-------|
| Thr 6–8 | Pro 3 | Val 7–8 | Ile 2 | | Arg 3 |
| Ser 4–5 | Gly 7 | Cys 4 | Leu 4 | His 2 | Trp 1 |

und der einen isoelektrischen Punkt bei 4,53 ± 0,15 besitzt oder bestehend aus den beiden Komponenten HOE 467-A und HOE 467-B erhalten wird.

2. Verfahren gemäss Anspruch 1, dadurch gekennzeichnet, das man den abgetrennten Inaktivator HOE 467 mit Hilfe von Ionenaustauschern im pH-Bereich zwischen 4,8 und 5,3 in die beiden Komponenten HOE 467-A und HOE 467-B auftrennt.

3. Verfahren gemäss Anspruch 1, dadurch gekennzeichnet, dass man den abgetrennten Inaktivator HOE 467 mit Hilfe von Ionenaustauschern im pH-Bereich zwischen 4,4 und 6 in die beiden Komponenten HOE 467-A und HOE 467-B auftrennt und HOE 467-A durch Chromatographie an DEAE- oder Carboxymethylcellulose-Säulen in Gegenwart eines dissoziierenden Puffers weiter reinigt und isoliert.

4. Verfahren gemäss Anspruch 1, 2 oder 3, dadurch gekennzeichnet, dass man den α-Amylaseinaktivator HOE 467-A, welcher ein Molekulargewicht von 7958 besitzt und die 74 Aminosäuren in folgender Sequenz aufweist:
Asp-Thr-Thr-Val-Ser-Glu-Pro-Ala-Pro-Ser-Cys-Val-Thr-Leu-Tyr-Gln-Ser-Trp-Arg-Tyr-Ser-Gln-Ala-Asp-Asn-Gly-Cys-Ala-Glu-Thr-Val-Thr-Val-Lys-Val-Val-Tyr-Glu-Asp-Asp-Thr-Glu-Gly-Leu-Cys-Tyr-Ala-Val-Ala-Pro-Gly-Gln-Ile-Thr-Thr-Val-Gly-Asp-Gly-Tyr-Ile-Gly-Ser-His-Gly-His-Ala-Arg-Tyr-Leu-Ala-Arg-Cys-Leu,
wobei zwischen Cys 11 und Cys 27 sowie zwischen Cys 45 und Cys 73 Disulfidbrücken sind, isoliert.

**Claims for the contracting states:**
**BE, CH, DE, FR, GB, LI, NL, SE**

1. An α-amylase inactivator comprising HOE 467-A, the hydrolysis product thereof having the following aminoacid composition:

| Asp 6 | Glu 7 | Ala 7 | | Tyr 6 | Lys 1 |
|-------|-------|-------|-------|-------|-------|
| Thr 8 | Pro 3 | Val 8 | Ile 2 | | Arg 3 |
| Ser 5 | Gly 7 | Cys 4 | Leu 4 | His 2 | Trp 1 |

and which has an isoelectric point at 4.35 ± 0.15, or comprising HOE 467-B the hydrolysis product thereof having the following aminoacid composition:

| Asp 5 | Glu 6–7 | Ala 7 | | Tyr 6 | Lys 1 |
|-------|---------|-------|-------|-------|-------|
| Thr 6–8 | Pro 3 | Val 7–8 | Ile 2 | | Arg 3 |
| Ser 4–5 | Gly 7 | Cys 4 | Leu 4 | His 2 | Trp 1 |

and which has an isoelectric point at 4.35 ± 0.15, or comprising the two components HOE 467-A and HOE 467-B.

2. An α-amylase inactivator as claimed in claim 1, wherein HOE 467-A has a molecular weight of 7,958 and the 74 aminoacids have the following sequence:
Asp-Thr-Thr-Val-Ser-Glu-Pro-Ala-Pro-Ser-Cys-Val-Thr-Leu-Tyr-Gln-Ser-Trp-Arg-Tyr-Ser-Gln-Ala-Asp-Asn-Gly-Cys-Ala-Glu-Thr-Val-Thr-Val-Lys-Val-Val-Tyr-Glu-Asp-Asp-Thr-Glu-Gly-Leu-Cys-Tyr-Ala-Val-Ala-Pro-Gly-Gln-Ile-Thr-Thr-Val-Gly-Asp-Gly-Tyr-Ile-Gly-Ser-His-Gly-His-Ala-Arg-Tyr-Leu-Ala-Arg-Cys-Leu,
and disulfide bridges allocated between Cys 11 and Cys 27 and betwee Cys 45 and Cys 73.

3. A process for the preparation of the α-amylase inactivator as claimed in claim 1, which comprises cultivating Streptomyces tendae ATCC 31210 or HAG 1266 and separating off the inactivator HOE 467 from the culture, with the aid of adsorption resins or reversed phase chromatography, and then separating the inactivator with the aid of ion exchanger chromatography in the pH range of 4.4 to 6 in the two components HOE 467-A and HOE 467-B.

4. A process as claimed in claim 3, wherein the inactivator HOE 467 which has been separated off, is separated with the aid of ion exchangers in the pH range between 4.8 and 5.3 into the two components HOE 467-A and HOE 467-B.

5. A process as claimed in claim 3, wherein the inactivator HOE 467 has been separated off, is separated with the aid of ion exchangers in the pH range between 4.4 and 6 into the two components HOE 467-A and HOE 467-B, and HOE 467-A is further purified and isolated by chromatography on DEAE- or carboxymethyl-cellulose columns, in the presence of a dissociating buffer.

6. Streptomyces tendae HAG 1266 (DSM 1912).

7. A pharmaceutical preparation containing the α-amylase inactivator as claimed in claim 1.

8. α-amylase inactivator as claimed in claim 1 for regulating the blood sugar level.

**Claims for the contracting state: AT**

1. A process for the preparation of an α-amylase inactivator, which comprises cultivating Streptomyces tendae ATCC 31210 or DSM 1912 and separating off the inactivator HOE 467 from the culture, with the aid of adsorption resins or reversed phase chromatography, and then separating the inactivator with the aid of ion exchanger chromatography in the pH range of 4.4 to 6 in the two components, whereby an α-amylase inactivator comprising HOE 467-A, the hydrolysis product thereof having the following aminoacid composition:

| Asp 6 | Glu 7 | Ala 7 | | Tyr 6 | Lys 1 |
|-------|-------|-------|-------|-------|-------|
| Thr 8 | Pro 3 | Val 8 | Ile 2 | | Arg 3 |
| Ser 5 | Gly 7 | Cys 4 | Leu 4 | His 2 | Trp 1 |

and which has an isoelectric point at 4.35 ± 0.15, or comprising the inactivator HOE 467-B the hydrolysis product thereof having the following aminoacid composition:

| Asp 5 | Glu 6–7 | Ala 7 | | Tyr 6 | Lys 1 |
|---|---|---|---|---|---|
| Thr 6–8 | Pro 3 | Val 7–8 | Ile 2 | | Arg 3 |
| Ser 4–5 | Gly 7 | Cys 4 | Leu 4 | His 2 | Trp 1 |

and which has an isoelectric point at 4.35 ± 0.15, or comprising the two components HOE 467-A and HOE 467-B is obtained.

2. A process as claimed in claim 1, wherein the inactivator HOE 467 which has been separated off, is separated with the aid of ion exchanges in the pH range between 4.8 and 5.3 into the two components HOE 467-A and HOE 467-B.

3. A process as claimed in claim 1, wherein the inactivator HOE 467 has been separated off, is separated with the aid of ion exchangers in the pH range between 4.4 and 6 into the two components HOE 467-A and HOE 467-B, and HOE 467-A is further purified and isolated by chromatography on DEAE- or carboxymethyl-cellulose columns, in the presence of a dissociating buffer.

4. A process as claimed in claim 1, 2 or 3 wherein the α-amylase inactivator HOE 467-A which has a molecular weight of 7,958 and the 74 aminoacids thereof have the following sequence:
Asp-Thr-Thr-Val-Ser-Glu-Pro-Ala-Pro-Ser-Cys-
Val-Thr-Leu-Tyr-Gln-Ser-Trp-Arg-Tyr-Ser-Gln-
Ala-Asp-Asn-Gly-Cys-Ala-Glu-Thr-Val-Thr-Val-
Lys-Val-Val-Tyr-Glu-Asp-Asp-Thr-Glu-Gly-Leu-
Cys-Tyr-Ala-Val-Ala-Pro-Gly-Gln-Ile-Thr-Thr-
Val-Gly-Asp-Gly-Tyr-Ile-Gly-Ser-His-Gly-His-
Ala-Arg-Tyr-Leu-Ala-Arg-Cys-Leu,
and disulfide bridges allocated between Cys 11 and Cys 27 and between Cys 45 and Cys 73 is isolated.

**Revendications pour les Etats contractants: BE, CH, DE, FR, GB, LI, NL, SE**

1. Inhibiteur d'alpha-amylase constitué de l'inhibiteur HOE 467-A dont l'hydrolysat présente la composition suivante:

| Asp 6 | Glu 7 | Ala 7 | | Tyr 6 | Lys 1 |
|---|---|---|---|---|---|
| Thr 8 | Pro 3 | Val 8 | Ile 2 | | Arg 3 |
| Ser 5 | Gly 7 | Cys 4 | Leu 4 | His 2 | Trp 1 |

et qui a un point isoélectrique de 4,35 ± 0,15, ou constitué de l'inhibiteur HOE 467-B dont l'hydrolysat à la composition suivante:

| Asp 5 | Glu 6–7 | Ala 7 | | Tyr 6 | Lys 1 |
|---|---|---|---|---|---|
| Thr 6–8 | Pro 3 | Val 7–8 | Ile 2 | | Arg 3 |
| Ser 4–5 | Gly 7 | Cys 4 | Leu 4 | His 2 | Trp 1 |

et qui a un point isoélectrique de 4,53 ± 0,15, ou constitué des deux constituants HOE 467-A et HOE 467-B.

2. Inhibiteur d'alpha-amylase selon la revendication 1, caractérisé en ce que HOE 467-A a une masse moléculaire de 7958, en ce que les 74 acides aminés montrent la séquence suivante:
Asp-Thr-Thr-Val-Ser-Glu-Pro-Ala-Pro-Ser-Cys-
Val-Thr-Leu-Tyr-Gln-Ser-Trp-Arg-Tyr-Ser-Gln-
Ala-Asp-Asn-Gly-Cys-Ala-Glu-Thr-Val-Thr-Val-
Lys-Val-Val-Tyr-Glu-Asp-Asp-Thr-Glu-Gly-Leu-
Cys-Tyr-Ala-Val-Ala-Pro-Gly-Gln-Ile-Thr-Thr-
Val-Gly-Asp-Gly-Tyr-Ile-Gly-Ser-His-Gly-His-
Ala-Arg-Tyr-Leu-Ala-Arg-Cys-Leu
et en ce que entre Cys 11 et Cys 27, ainsi qu'entre Cys 45 et Cys 73, se trouvent des ponts disulfure.

3. Procédé pour la préparation de l'inhibiteur d'alpha-amylase selon la revendication 1, caractérisé en ce qu'on cultive Streptomyces tendae ATCC 31210 ou HAG 1266 et à partir de la culture on isole l'inhibiteur HOE 467 au moyen de résines d'adsorption ou par chromatographie en phase inverse, puis par chromatographie par échange d'ions dans un domaine de pH de 4,4 à 6 on sépare en les deux constituants HOE 467-A et HOE 467-B.

4. Procédé selon la revendication 3, caractérisé en ce que, au moyen d'échangeurs d'ions dans un domaine de pH entre 4,8 et 5,3, on sépare l'inhibiteur HOE 467 isolé en les deux constituants HOE 467-A et HOE 467-B.

5. Procédé selon la revendication 3, caractérisé en ce que, au moyen d'échangeurs d'ions dans un domaine de pH de 4,4 à 6, on sépare l'inhibiteur HOE 467 isolé en les deux constituants HOE 467-A et HOE 467-B, puis on purifie et on isole HOE 467-A par chromatographie sur des colonnes de DEAE-cellulose ou de carboxyméthyl-cellulose en présence d'un tampon se dissociant.

6. Streptomyces tendae HAG 1266 (DSM 1912).

7. Composition pharmaceutique contenant l'inhibiteur d'alpha-amylase selon la revendication 1.

8. Inhibiteur d'alpha-amylase selon la revendication 1 pour la régulation du taux de glycémie dans le sang.

**Revendications pour Etat contractant: AT**

1. Procédé pour la préparation d'un inhibiteur d'alpha-amylase, caractérisé en ce qu'on cultive Streptomyces tendae ATCC 31210 ou DSM 1912, puis à partir de la culture on isole l'inhibiteur HOE 467 au moyen de résines d'adsorption ou par chromatographie en phase inverse, et, finalement par chromatographie par échange d'ions dans un domaine de pH de 4,4 à 6, on sépare en deux constituant, et on obtient.un inhibiteur d'alpha-amylase constitué de HOE 467-A dont l'hydrolysat montre la composition en acides aminés suivante:

| Asp 6 | Glu 7 | Ala 7 | | Tyr 6 | Lys 1 |
|---|---|---|---|---|---|
| Thr 8 | Pro 3 | Val 8 | Ile 2 | | Arg 3 |
| Ser 5 | Gly 7 | Cys 4 | Leu 4 | His 2 | Trp 1 |

et qui a un point isoélectrique de 4,35 ± 0,15, ou constitué de l'inhibiteur HOE 467-B dont l'hydrolysat montre la composition en acides aminés suivante:

| Asp 5 | Glu 6–7 | Ala 7 | | Tyr 6 | Lys 1 |
|---|---|---|---|---|---|
| Thr 6–8 | Pro 3 | Val 7–8 | Ile 2 | | Arg 3 |
| Ser 4–5 | Gly 7 | Cys 4 | Leu 4 | His 2 | Trp 1 |

et qui a un point isoélectrique de 4,53 ± 0,15, ou constitué des deux constituants HOE 467-A et HOE 467-B.

2. Procédé selon la revendication 1, caractérisé en ce que, au moyen d'échangeurs d'ions dans un domaine de pH entre 4,8 et 5,3, on sépare l'inhibiteur HOE 467 isolé en les deux constituants HOE 467-A et HOE 467-B.

3. Procédé selon la revendication 1, caractérisé en ce que, au moyen d'échangeurs d'ions dans un domaine de pH entre 4,4 et 6, on sépare l'inhibiteur HOE 467 isolé en les deux constituants HOE 467-A et HOE 467-B et on purifie et on isole HOE 467-A par chromatographie sur des colonnes de DEAE-cellulose ou de carboxyméthyl-cellulose en présence d'un tampon se dissociant.

4. Procédé selon l'une quelconque des revendications 1, 2 ou 3, caractérisé en ce que on isole l'inhibiteur d'alpha-amylase HOE 467-A qui a une masse moléculaire de 7958 et montre les 74 acides aminés dans la séquence suivante:
Asp-Thr-Thr-Val-Ser-Glu-Pro-Ala-Pro-Ser-Cys-Val-Thr-Leu-Tyr-Gln-Ser-Trp-Arg-Tyr-Ser-Gln-Ala-Asp-Asn-Gly-Cys-Ala-Glu-Thr-Val-Thr-Val-Lys-Val-Val-Tyr-Glu-Asp-Asp-Thr-Glu-Gly-Leu-Cys-Tyr-Ala-Val-Ala-Pro-Gly-Gln-Ile-Thr-Thr-Val-Gly-Asp-Gly-Tyr-Ile-Gly-Ser-His-Gly-His-Ala-Arg-Tyr-Leu-Ala-Arg-Cys-Leu,
des ponts disulfure se trovant entre Cys 1 et Cys 27, ainsi qu'enter Cys 45 et Cys 73.